# EUROPEAN PATENT SPECIFICATION

(11) **EP 4 056 206 B1**
(45) Date of publication and mention of the grant of the patent: **01.01.2025**
(21) Application number: 21162122.2
(22) Date of filing: 11.03.2021
(51) Int. Cl.: A61L 27/22, A61L 27/24, A61L 27/38, A61L 27/36, A61L 27/52

(54) **ARTIFICIAL ENDOTHELIAL KERATOPLASTY GRAFT AND METHODS OF PREPARATION THEREOF**
KÜNSTLICHES ENDOTHELIALES KERATOPLASTIE-TRANSPLANTAT UND VERFAHREN ZU DESSEN HERSTELLUNG
GREFFE PAR KÉRATOPLASTIE ENDOTHÉLIALE ARTIFICIELLE ET SES MÉTHODES DE PRÉPARATION

(43) Date of publication of application: 14.09.2022
(73) Proprietor: Precise Bio Inc., Winston Salem, North Carolina 27101 (US)
(72) Inventor: BATT, Aryeh, 9044000 Har Hebron (IL); EITAN, Amos, 93123 Jerusalem (IL); EISENBACH, Ariel, 44865 Zufim (IL); HAYARDENI, Yishay, Greensboro, NC 27410 (US); KHAN, Mehwish, High Point, NC 27262 (US); MARCUS, Michal, 4995000 Nechalim (IL); NEPAL, Kanti, Winston Salem, NC 27107 (US); NGUYEN, Khanh, Winston Salem, NC 27104 (US); SHAV, Lior, Glen Rock, NJ 07452 (US); WU, Yan, Winston Salem, NC 27101 (US)
(74) Representative: Vossius & Partner Patentanwälte Rechtsanwälte mbB

(56) References cited:
- EP-A1- 2 468 312
- EP-A1- 2 483 332
- EP-A1- 2 755 598
- EP-A1- 2 940 126
- EP-B1- 2 483 332
- EP-B1- 2 755 598
- WO-A1-2007/043255
- WO-A1-2019/198086
- RIZWAN MUHAMMAD ET AL: "Sequentially-crosslinked bioactive hydrogels as nano-patterned substrates with customizable stiffness and degradation for corneal tissue engineering applications", BIOMATERIALS, ELSEVIER, AMSTERDAM, NL, vol. 120, 23 December 2016 (2016-12-23), pages 139 - 154, XP029879408, ISSN: 0142-9612, DOI: 10.1016/J.BIOMATERIALS.2016.12.026
- AN JEONG-HEE ET AL: "Tissue engineered ultra-thin descemet stripping corneal endothelial layers using porcine cornea and stem cells", EXPERIMENTAL EYE RESEARCH, ACADEMIC PRESS LTD, LONDON, vol. 199, 14 August 2020 (2020-08-14), XP086286432, ISSN: 0014-4835, [retrieved on 20200814], DOI: 10.1016/J.EXER.2020.108192

## Description

### FIELD OF INVENTION

The present invention generally pertains to an artificial endothelial keratoplasty graft and to methods of its preparation.

### BACKGROUND OF THE INVENTION

Corneal blindness is estimated to have effect on the order of 10 million people worldwide. It is further estimated that only about 1% of the people suffering from corneal blindness receive treatment. In part, the low rate of treatment is due to the scarcity of corneal tissue donors. Even in cases in which corneal tissue is available, problems frequently remain, as corneal tissue can easily be damaged during the transplantation procedure, and tends to be less healthy in older donors than in younger ones. Furthermore, the time frame for the donation is quite short, as the tissue should be harvested within several hours of death and transplanted within a few days after harvesting.

The term corneal transplantation is used to describe several different medical procedures, in which different parts of the cornea are implanted, including: (A) Penetrating Keratoplasty (PK), in which the entire thickness of the cornea is transplanted, (B) Descemet Stripping Endothelial Keratoplasty (DSEK), in which the endothelium membrane, Descemet membrane and part of the posterior corneal stroma are transplanted, and (C) Descemet Membrane Endothelial Keratoplasty (DMEK), in which the endothelium and the Descemet membrane are transplanted, without an additional stroma layer.

Among the procedures mentioned above, DSEK is the most common one. In the US, 60% of the total corneal transplantations are DSEK. However, the DMEK procedure has major advantages over it, including lower rejection rate (1% for DMEK compared with 12% of DSEK), higher probability for visual acuity above 0.8 (79% in DMEK compared with 47% in DSEK), and a shorter healing period (weeks in DMEK compared with months to a year in DSEK).

The main reason that the DMEK procedure is not fully taking over the DSEK is the ease of implantation. In particular, the native DMEK graft tends to break more easily during the procedure, and the endothelial cells in it are sensitive to pressure and shear stresses. Therefore, the DMEK usually requires special implantation techniques and higher experienced surgeons.

In addition, since the DMEK is so fragile and sensitive, doctors prefer a stiffer graft obtained from donors older than 50 or 60 years. However, the corneal endothelial cells in these grafts are lower in density and conditions.

Thus, a reliable source for thin yet stiff corneal implants, with healthy and functioning endothelial cells and repeatable conditions, remains an unmet need.

Publications disclosing a method for the preparation of endothelial keratoplasty grafts include EP2940126; EP2468312; EP2483332; and Rizwan, Muhammad et al. "Sequentially-crosslinked bioactive hydrogels as nano-patterned substrates with customizable stiffness and degradation for corneal tissue engineering applications", Biomaterials, Elsevier, Amsterdam, NL, vol. 120, 23 December 2016, pages 139-154.

### BRIEF DESCRIPTION OF THE DRAWINGS

The various features and advantages of the present invention may be more readily understood with reference to the following detailed description taken in conjunction with the accompanying drawings in which
**Fig. 1** schematically illustrates a DMEK-like graft according to one embodiment of the invention;
**Fig. 2** schematically illustrates a DMEK-like graft on a designated carrier according to another embodiment of the invention; and
**Fig. 3** schematically illustrates a corneal graft orientation in the eye, according to yet another embodiment of the invention.

### SUMMARY OF THE INVENTION

It is thus an object of the current invention to disclose the preparation of PVEK (Precise Vision Endothelial Keratoplasty), namely a DMEK-like implant, which may be implanted using the detachable and/or dissolvable carrier as claimed.

The terms "Precise vision endothelial keratoplasty" and PVEK, will be used interchangeably herein after refer to a set of solutions found in a few cases being equal to, if not better than the DSEK implant, as disclosed in US Pat. Appl. No. 62/487,018 "Bioengineered corneal graft and methods of preparation thereof.

The PVEK-implant of the present invention is very thin, but is still stiff enough to handle with standard ophthalmic tools. Also, native DMEK grafts tend to fold due to the contraction of the cells on the Descemet membrane. This makes the implantation much more difficult as the surgeon has to flatten the graft in the eye. Here however, the membrane does not tend to fold and the graft opens more easily after insertion.

The PVEK-grafting by the present technology comprises, *inter alia,* step(s) of drying out a solution of collagen on a surface, and crosslinking it with EDC/NHS solution, that bond the collagen polymer and make it a hydrogel thin film. Since the crosslinking process occurs while the material is not fully wetted, the resulting hydrogel is very thin and has a high polymer concentration. These layers can be implanted in a similar way to DMEK, but have an advantage in that they do not tend to fold after implantation like DMEK. The present invention hence discloses, *inter alia,* an artificial endothelial keratoplasty graft according to claim 1.

Further developments of the invention are according to claims 2-4.

The present invention further discloses a method of manufacturing an artificial endothelial keratoplasty graft, according to claim 5.

Further developments of the invention are according to claims 6-10.

### DETAILED DESCRIPTION OF THE PREFERRED EMBODIMENTS

The artificial Endothelial Keratoplasty Implant and methods of preparation thereof disclosed herein are designed to meet the aforementioned needs, by allowing the production of DMEK-like grafts with healthy and functioning cells and good mechanical properties.

The implant is made of Descemet membrane-like hydrogel support layer, and a layer of corneal endothelial cells (CEC) on top. The endothelial layer is made of CEC cells obtained from donors' corneas and proliferated, or by stem-cells differentiation, by methods known in the art.

The support layer is made of a thin collagen layer, designed to have mechanical properties, transparency and dimensions to improve patients' vision and health condition.

To produce the endothelial keratoplasty, a layer of low concentration collagen solution is spread on a surface and dehydrated. After drying out, a crosslinker solution is introduced to the dried material, which forms a crosslinked hydrogel in a thicknesses of 2 to 50 micrometers. The excess and un-crosslinked reagents are washed away using PBS and corneal endothelial cells are seeded on top of the hydrogel, forming a two-layer construct, similarly to DMEK cadaver grafts.

In some cases, the corneal graft is implanted onto the posterior cornea using a DMEK tool or carrier. In preferred embodiments, prior to the implantation, a carrier layer is attached to the corneal graft using a biocompatible adhesive material, forming a 4-layer implant: An Endothelial keratoplasty graft made of a collagen layer and a cells layer, an adhesive layer, and a carrier. After implantation, the adhesive and the carrier layer are possibly detached from the graft and pulled out or dissolved in the eye.

In other cases, the corneal graft is implanted similarly to cadaver-sourced DMEK grafts, by methods known in the art.

The present invention hence discloses, *inter alia,* an artificial endothelial keratoplasty graft comprising a support layer made of rehydrated crosslinked hydrogel and corneal endothelial cells on top or within said support layer.

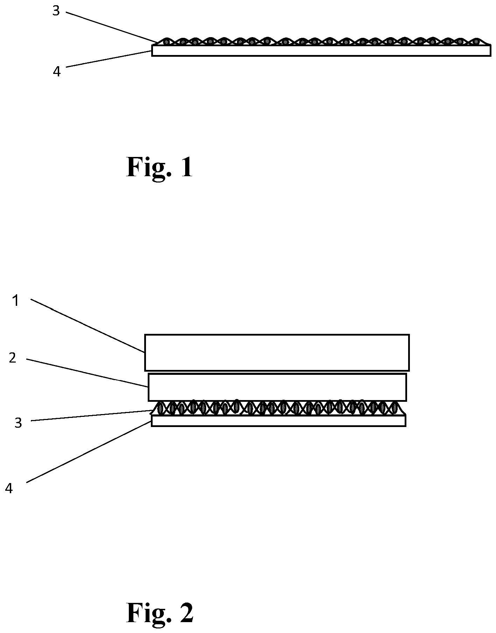

Reference is made to **figure 1****,** schematically illustrating a DMEK-like graft according to one embodiment of the invention. An upper layer is corneal endothelial cells (3) and below is a support layer (4).

Reference is now made to **figure 2****,** schematically illustrating a DMEK-like graft on a designated carrier according to another embodiment of the invention. The upper layer in this illustration is a carrier (1); and below it are an adhesive (2), corneal endothelial cells (3), and support layer (4).

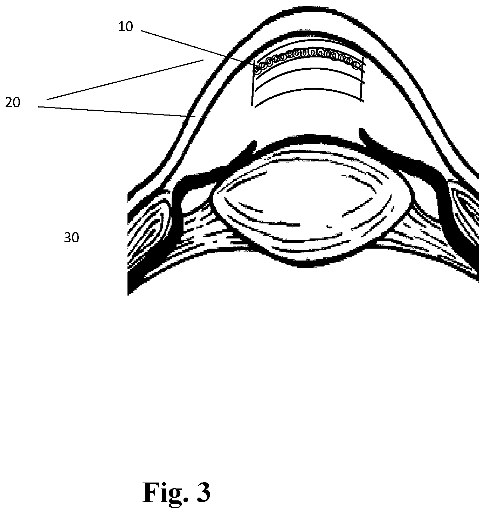

Reference is now made to **figure 3****,** schematically illustrating a corneal graft orientation in the eye, according to yet another embodiment of the invention. Upper illustration is a corneal graft with adhesive and carrier layers attached to the posterior cornea (not in scale) (10). Cornea (20) and anterior chamber (30) are also illustrated. Figure is adapted from a currently available public draw https://www.iconspng.com/image/92595/eye-3.

According to a few embodiments of the invention, the graft comprises two main layers, namely *(i)* a support (Descemet membrane-like) layer, made of collagen or collagen methacrylate; and *(ii),* a corneal endothelial cells layer, seeded on top of the support layer

According to a few embodiments of the invention, the geometry of the support layer ranges between about 2 and about 50 microns thick; the diameter ranging between about 7.5 to about 9.5 mm.

According to a few other embodiments of the invention, the geometry of the cells layer ranges between about 2 and about 20 microns in thickness, and a cell density of about 1,500 to about 4,000 cells/mm²; and a diameter of about 7.5 mm to about 9.5 mm.

According to the invention, the materials of the support layer are selected from collagen or collagen methacrylate, (from about 1 to about 50% w/v., whereas native stroma has 13% collagen).

According to a few embodiments of the invention, additional materials of the support layer are photo-initiators and crosslinkers selected from the group consisting of: lithium phenyl-2,4,6-trimethylbenzoylphosphinate (LAP), 1-[4-(2-Hydroxyethoxy)-phenyl]-2-hydroxy-2-methyl-1-propane-1-one (Irgacure 2959), 1-ethyl-3-(3-dimethylaminopropyl) carbodiimide (EDC), N-hydroxysuccinimide (NHS), and a combination thereof.

According to a few embodiments of the invention, additional materials of the support layer are selected from the group consisting of biocompatible dyes for easy handling, such as trypan blue.

According to a few embodiments of the invention, the materials of the cells layer are selected from the group consisting of (i) human corneal endothelial cells from human donors, with or without expansion of the cells by a proliferation step; (ii) human corneal endothelial cells derived from stem cells such as induced pluripotent stem cells (iPSC) or human embryonic stem cells (hESC); (iii) the cells' own ECM, produced by the cells during the maturation process and any combination thereof.

According to a few embodiments of the invention, the preparation methods comprise drying step(s). Such drying steps comprise the steps of spreading or molding about 0.1 to about 15% (w/v) materials selected from collagen, and collagen methacrylate, on a surface. The spread/molded layer thickness may vary between about 0.1 and about 10 mm. Then, the materials are allowed to dry at a specific range of temperatures, between about 4 and 60 degrees Celsius, and relative humidity levels. The total drying time ranges from 1 hour to about 10 days. The drying process may consist of several different environmental condition steps.

According to the invention, the preparation methods comprise crosslinking step(s). After drying, there is the step of crosslinking the dried sheet by washing it with one or more of the following: EDC, NHS, LAP, 1-[4-(2-Hydroxyethoxy)-phenyl]-2-hydroxy-2-methyl-1-propane-1-one or other crosslinker solution, or a mixture of the above. Using a photo-initiating light source is possible to induce the process. The crosslinking time is between about 1 minute and about 48 hours. Afterwards, there is the step of washing the crosslinked gel to remove residues of crosslinker and un-crosslinked polymer, and immersing the crosslinked gel in water/PBS/media. Then, possibly repeating the drying and crosslinking steps multiple times.

The present invention discloses a method of manufacturing an artificial endothelial keratoplasty graft, the method consisting of a step of drying a support layer material followed by a crosslinking step, wherein the support layer material is collagen or collagen methacrylate.

According to a few embodiments of the invention, the preparation methods comprise seeding step(s), namely seeding corneal endothelial cells on top of the gel; and maturing for about 1 day to about 28 days in an incubator.

### EXAMPLE 1

Spreading 160µL of 0.5% (w/v) collagen on an area of 10mm×10mm, on a flat surface (e.g. Petri dish), to form a layer of about 2 mm thick. Letting the material dry at 4 degrees Celsius, 40% RH, for 48 hours. Adding 2ml PBS solution with (1 mg/ml) EDC and (0.25mg/ml) NHS, and letting the material crosslink for 2 h at 25 degrees Celsius. Rinsing the gel 3 times in PBS, with a final wash of 12 hours in PBS. Pipetting out the PBS and seeding CECs on the hydrogel surface.

REFERENCE EXAMPLE (not falling within the scope of the claimed subject-matter).

Spreading 100µL of 5% (w/v) gelatin methacrylate on an area of 10 mm × 10 mm, on a flat hydrophobic plastic surface (e.g. Petri dish), to form a layer of about 1.2 mm thick. Letting the material dry in a 25 degrees Celsius vacuum desiccator for 12 hours. Adding 2ml PBS solution with (0.5% w/v) LAP, and applying UV light at 1 mW/cm^2 to crosslink the material for 30 minutes. Rinsing the gel 3 times in PBS, with a final wash of 12 hours in PBS. Pipetting out the PBS and seeding CECs on the hydrogel surface. It is within the scope of the present invention to provide the embodiments as disclosed in the following examples (examples 3-6, as outlined below).

Grafts are implanted via a few optional procedures, see examples 3-4, which are not falling within the scope of the claimed subject-matter.

### REFERENCE EXAMPLE 3

The artificial EK graft was implanted using standard DMEK or DSEK techniques and tools, such as a Coronet Endoglide (commercially available by Network Medical Products) or a Geuder cannula. This included loading the graft onto the tool (before or after shipment), inserting the tool through a peripheral corneal incision, injecting/pulling/pushing the graft into the patient's anterior chamber, and attaching the graft to the posterior cornea with an air bubble.

### PREFERENCE EXAMPLE 4

To make the implantation procedure easier and safer, a designated carrier device was used to insert the graft into the patient's eye (see **Fig. 2**). The carrier was made of detachable/dissolvable thermo-responsive materials, with a diameter similar to the graft or slightly larger. The EK graft was attached to the carrier device, folded and inserted into the eye. After insertion, an air bubble was injected to attach the graft to the posterior cornea. Then, the cornea was warmed to 34-38 degrees Celsius to detach or dissolve the carrier.

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably. The expression "of any of claims XX-YY" (wherein XX and YY refer to claim numbers) is intended to provide a multiple dependent claim in the alternative form, and in some embodiments is interchangeable with the expression "as in any one of claims XX-YY."

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are described. Nothing herein is to be construed as an admission that the invention is not entitled to antedate such disclosure by virtue of prior invention.

Whenever a range is given in the specification, for example, a range of integers, a temperature range, a time range, a composition range, or concentration range, all intermediate ranges and subranges, as well as all individual values included in the ranges given are intended to be included in the disclosure. As used herein, ranges specifically include the values provided as endpoint values of the range. As used herein, ranges specifically include all the integer values of the range. For example, a range of 1 to 100 specifically includes the end point values of 1 and 100. It will be understood that any subranges or individual values in a range or subrange that are included in the description herein can be excluded from the claims herein. The term "about" refers to any value being lower or greater than 20% of the defined measure.

As used herein, "comprising" is synonymous and can be used interchangeably with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. In each instance herein any of the terms "comprising", "consisting essentially of" and "consisting of" can be replaced with either of the other two terms. The invention illustratively described herein suitably can be practiced in the absence of any element or elements, limitation or limitations which is not specifically disclosed herein.

## Claims

1. An artificial endothelial keratoplasty graft comprising a support layer made of crosslinked hydrogel and corneal endothelial cells on top or within said support layer; **characterized in that** said hydrogel was initially dried and only then crosslinked, wherein the crosslinked hydrogel is based on crosslinked collagen or collagen methacrylate.

2. The graft of claim 1, wherein the source of said collagen or collagen methacrylate is human recombinant collagen.

3. The graft of any one of claims 1 to 2, wherein the total thickness of the graft is between 5 and 50 microns or below 25 microns, therefore mimicking native DMEK (Descemet Membrane Endothelial Keratoplasty) graft.

4. The graft of any one of claims 1 to 3, wherein said corneal endothelial cells are configured to remodel said support layer to form corneal endothelium extra-cellular matrix, via a long maturation period or by additional nutrition to the cells.

5. A method of manufacturing an artificial endothelial keratoplasty graft, the method comprising:
drying a support layer material; and crosslinking after said drying,
wherein the support layer material is collagen or collagen methacrylate.

6. The method of claim 5, wherein the source of said collagen or collagen methacrylate is human recombinant collagen.

7. The method of any one of claims 5 and 6, wherein the crosslinking step involves introducing photo-initiators and crosslinkers selected from the group consisting of 1-ethyl-3-(3-dimethylaminopropyl) carbodiimide (EDC) and N-hydroxysuccinimide (NHS) molecules to the material, and/or introducing lithium phenyl-2,4,6-trimethylbenzoylphosphinate (LAP) or 1-[4-(2-hydroxyethoxy)-phenyl]-2-hydroxy-2-methyl-1-propane-1-one (Irgacure 2959) molecules to the support layer material and applying light on it.

8. The method of any one of claims 5 to 7, wherein the water content in said support layer material is between 30 and 90 percent.

9. The method of any one of claims 5 to 8, wherein the drying step is performed at between 4 and 60 degrees Celsius.

10. The method of any one of claims 5 to 9, wherein the final thickness of said graft is below 25 microns.

## Patentansprüche

1. Künstliches endotheliales Keratoplastik-Trans- und/oder Implantat, das eine Trägerschicht aus vernetztem Hydrogel und Hornhautendothelzellen auf oder innerhalb der Trägerschicht aufweist; **dadurch gekennzeichnet, dass** das Hydrogel zunächst getrocknet und erst dann vernetzt wurde, wobei das vernetzte Hydrogel auf vernetztem Kollagen oder Kollagenmethacrylat basiert.

2. Trans- und/oder Implantat nach Anspruch 1, wobei die Quelle des Kollagens oder Kollagenmethacrylats humanes rekombinantes Kollagen ist.

3. Trans- und/oder Implantat nach einem der Ansprüche 1 bis 2, wobei die Gesamtdicke des Trans- und/oder Implantats zwischen 5 und 50 Mikrometer oder unter 25 Mikrometer liegt und somit ein natives DMEK (Descemetmembran-Endotheliale-Keratoplastik) Transplantat imitiert.

4. Trans- und/oder Implantat nach einem der Ansprüche 1 bis 3, wobei die Hornhautendothelzellen konfiguriert sind, die Trägerschicht zu remodellieren, um eine extrazelluläre Hornhautendothelmatrix zu bilden, über eine lange Reifungsperiode oder durch zusätzliche Ernährung der Zellen.

5. Verfahren zur Herstellung eines künstlichen endothelialen Keratoplastik-Trans- und/oder Implantats, wobei das Verfahren aufweist:
Trocknen eines Trägerschichtmaterials; und Vernetzen nach dem Trocknen, wobei das Trägerschichtmaterial Kollagen oder Kollagenmethacrylat ist.

6. Verfahren nach Anspruch 5, wobei die Quelle des Kollagens oder Kollagenmethacrylats humanes rekombinantes Kollagen ist.

7. Verfahren nach einem der Ansprüche 5 und 6, wobei der Vernetzungsschritt das Einbringen von Photoinitiatoren und Vernetzern, die aus der Gruppe ausgewählt sind, die aus 1-Ethyl-3-(3-dimethylaminopropyl)carbodiimid (EDC) und N-Hydroxysuccinimid (NHS)-Molekülen besteht, in das Material, und/oder Einbringen von Lithiumphenyl-2,4,6-trimethylbenzoylphosphinat (LAP) oder 1-[4-(2-Hydroxyethoxy)-phenyl]-2-hydroxy-2-methyl-1-propan-1-on (Irgacure 2959) Molekülen in das Trägerschichtmaterial und Beleuchten desselben umfasst.

8. Verfahren nach einem der Ansprüche 5 bis 7, wobei der Wassergehalt in dem Trägerschichtmaterial zwischen 30 und 90 % liegt.

9. Verfahren nach einem der Ansprüche 5 bis 8, wobei der Trocknungsschritt bei einer Temperatur zwischen 4 und 60 Grad Celsius durchgeführt wird.

10. Verfahren nach einem der Ansprüche 5 bis 9, wobei die Enddicke des Trans- und/oder Implantats unter 25 Mikrometer liegt.

## Revendications

1. Greffon de kératoplastie endothéliale artificielle comprenant une couche de support constituée d'hydrogel réticulé et de cellules endothéliales cornéennes au-dessus ou à l'intérieur de ladite couche de support ;
**caractérisé en ce que** ledit hydrogel a été initialement séché et seulement ensuite réticulé,
dans lequel l'hydrogel réticulé est à base de collagène ou de méthacrylate de collagène réticulé.

2. Greffon selon la revendication 1, dans lequel la source dudit collagène ou méthacrylate de collagène est du collagène recombinant humain.

3. Greffon selon l'une quelconque des revendications 1 à 2, dans lequel l'épaisseur totale du greffon est entre 5 et 50 microns ou inférieure à 25 microns, imitant ainsi le greffon DMEK (kératoplastie endothéliale de la membrane de Descemet) natif.

4. Greffon selon l'une quelconque des revendications 1 à 3, dans lequel lesdites cellules endothéliales cornéennes sont configurées pour remodéliser ladite couche de support pour former une matrice extracellulaire de l'endothélium cornéen, via une longue période de maturation ou par apport nutritionnel supplémentaire aux cellules.

5. Procédé de fabrication d'un greffon de kératoplastie endothéliale artificielle, le procédé comprenant :
le séchage d'un matériau de couche de support ; et une réticulation après ledit séchage,
dans lequel le matériau de couche de support est du collagène ou du méthacrylate de collagène.

6. Procédé selon la revendication 5, dans lequel la source dudit collagène ou méthacrylate de collagène est du collagène recombinant humain.

7. Procédé selon l'une quelconque des revendications 5 et 6, dans lequel l'étape de réticulation implique l'introduction de photo-initiateurs et d'agents de réticulation choisis dans le groupe constitué de molécules de 1-éthyl-3-(3-diméthylaminopropyl)carbodiimide (EDC) et de N-hydroxysuccinimide (NHS) dans le matériau, et/ou l'introduction de molécules de phényl-2,4,6-triméthylbenzoylphosphinate de lithium (LAP) ou de 1-[4-(2-hydroxyéthoxy)-phényl]-2-hydroxy-2-méthyl-1-propane-1-one (Irgacure 2959) dans le matériau de couche de support et l'application de lumière sur celui-ci.

8. Procédé selon l'une quelconque des revendications 5 à 7, dans lequel la teneur en eau dans ledit matériau de couche de support est entre 30 et 90 pour cent.

9. Procédé selon l'une quelconque des revendications 5 à 8, dans lequel l'étape de séchage est effectuée entre 4 et 60 degrés Celsius.

10. Procédé selon l'une quelconque des revendications 5 à 9, dans lequel l'épaisseur finale dudit greffon est inférieure à 25 microns.
